# EUROPEAN PATENT APPLICATION

(11) **EP 1 157 694 A1**
(43) Date of publication of application: **28.11.2001**
(21) Application number: 99973258.9
(22) Date of filing: 30.11.1999
(51) Int. Cl.: A61K 31/50, A61K 31/501, A61P 15/10, C07D 237/22

(54) **REMEDIAL AGENT FOR ERECTILE DYSFUNCTION**

(30) Priority: 07.12.1998 JP 346798
(71) Applicant: Nissan Chemical Industries, Ltd., Chiyoda-ku, Tokyo 101-0054 (JP); Welfide Corporation, Osaka-shi, Osaka 541-0046 (JP)
(72) Inventor: TANIKAWA, Keizo, Nissan Chem.Ind., Ltd., Funabaschi, Chiba 274-8507 (JP); TSURUZOE, Nobutomo, Nissan Chem. Industries, Ltd., Minamisaitama-gun, Saitama 349-0218 (JP); SHUDO, Norimasa, Nissan Chemical Industries, Ltd., Minamisaitama-gun, Saitama 349-0218 (JP); YAMASHITA, Toru, Nissan Chemical Industries, Ltd., Minamisaitama-gun, Saitama 349-0218 (JP); ISHIWATA, Norihisa, Nissan Chemical Industr., Ltd., Minamisaitama-gun, Saitama 349-0218 (JP); KIDO, Hideaki, Welfide Corporation, Chikujo-gun, Fukuoka 871 (JP); EBISU, Hajime, Welfide Corporation, Hirakata-shi, Osaka 573-1153 (JP); HAYASHI, Kazutaka, Welfide Corporation, Hirakata-shi, Osaka 573-1153 (JP); KUBO, Yoshiji, Welfide Corporation, Hirakata-shi, Osaka 573-1153 (JP); NAKAMURA, Norifumi, Welfide Corporation, Osaka-shi, Osaka 541-0046 (JP)
(74) Representative: Henkel, Feiler, Hänzel
(86) International application number: JP9906693
(87) International publication number: WO0033845

(57) **Abstract**

The present invention provides a remedial agent for erectile dysfunction which comprises a 3(2H)-pyridazinone derivative represented by the formula (I): 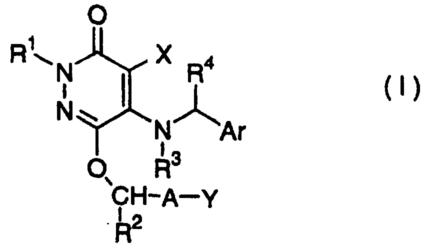 wherein each symbol is the same as defined in the specification
or a pharmaceutically acceptable salt thereof as an active ingredient.

## Description

### Technical field

The present invention relates to the use of a 3(2H)-pyridazinone derivative having pharmaceutical activity for the treatment of erectile dysfunction.

### Background art

In Japanese Unexamined Patent Publication No. Sho 63-301870 (US Patent No. 4,978,665, European Patent No. 275997B), Japanese Patent Publication No. Hei 7-107055 (US Patent No. 5,314,883, European Patent No. 482208B) and Japanese Unexamined Patent Publication No. Hei 7-252237 (US Patent No. 5,750,523, European Patent No. 742211A), preventive or therapeutic applications of the 3(2H)-pyridazinone derivative of the compound of the present invention has been disclosed as a platelet aggregation inhibitor, cardiotonic drug, vasodilator or anti-SRS-A (Slow Reacting Substances Anaphylaxis) drug for the prevention or treatment of various thrombotic diseases, ischemic diseases, hypertension, asthma or immediate allergic diseases and so forth, but there is no suggestion or mention whatsoever of the potential for application to the treatment of erectile dysfunction.

In addition, it has also been mentioned that the 3(2H)-pyridazinone derivative of the compound of the present invention also has both of phosphodiesterase III (PDE III) inhibitory activity and phosphodiesterase V (PDE V) inhibitory activity in platelets (Jpn. J. Pharmacol., vol. 67 Suppl. I, p. 204 (1995)).

However, there is no mention of the inhibitory activity on these enzymes in the corpus cavernosum.

On the other hand, specific PDE III inhibitors or specific PDE V inhibitors have been reported to be effective as therapeutic agents for the treatment of erectile dysfunction (Int. J. Impotence Res., vol. 4 Suppl. 2, p: 11 (1992), J. Urol., vol. 152, pp. 2159-2163 (1994), J. Urol., vol. 159, pp. 1390-1393 (1994), etc.). However, there is no suggestion nor mention whatsoever of the case of concomitant use of specific PDE III inhibitors and specific PDE V inhibitors, or of drugs having both PDE III inhibitory activity and PDE V inhibitory activity.

The inventors of the present invention unexpectedly found that the compound according to the present invention is useful in the treatment of erectile dysfunction, and that usefulness is improved as compared with treatment methods of the prior art, thereby leading to completion of the present invention.

Erectile dysfunction is caused by aging, stress or such illnesses as diabetes and hyperlipidemia, and is a disease that is characterized by failure to obtain or sustain an adequate erection for sexual intercourse. Its morbidity rate is increasing annually, and for example, it is estimated that there are 20 million men suffering from erectile dysfunction in the US alone.

There are various methods for treating erectile dysfunction, and drug therapy is one of its established treatment methods.

For example, a method is employed in which prostaglandin E1 is administered intracavernously or intraurethrally. In the case of intracavernous injection, although treatment is both fast-acting and has excellent efficacy, it is accompanied by pain in the penis and persistent penile rigidity. In addition, although intraurethral administration solves the problems associated with intracavernous injection, it invites a reduction in efficacy. Moreover, these treatment methods have serious problems in terms of being expensive and involving problems in convenience.

In addition, although specific PDE III inhibitors typically represented by milrinone are expected to be able to be used as remedial agents for erectile dysfunction, they have the problem of it being difficult to dissociate them from adverse side effects on the circulatory system such as the heart and blood vessels.

Moreover, although PDE V inhibitors typically represented by zaprinast and sildenafil are effective against organic erectile dysfunction, they have the problem of being ineffective against psychogenic erectile dysfunction. In addition, practically developed sildenafil has been reported to be associated with adverse side effects such as visual disorders.

In consideration of the present circumstances as described above, there is a need for a drug having both superior efficacy and safety for use as a remedial agent for erectile dysfunction. Thus, the object of the present invention is to provide a superior remedial agent for erectile dysfunction.

### Disclosure of the invention

The present invention relates to a remedial agent for erectile dysfunction comprising a 3(2H)-pyridazinone derivative represented by the formula (I): 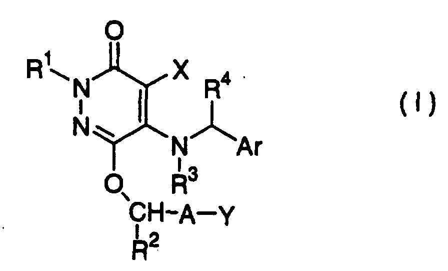
wherein R¹ represents a hydrogen atom, a C₁₋₄ alkyl group, a C₃₋₄ alkenyl group or (CH₂)ₙCO₂R⁵, n represents an integer of 1 to 4 and R⁵ represents a hydrogen atom or a C₁₋₄ alkyl group;
R² represents a hydrogen atom or a C₁₋₄ alkyl group;
R³ and R⁴ each independently represents a hydrogen atom or a C₁₋₃ alkyl group;
A represents a single bond or a straight or branched C₁₋₁₁ alkylene in which a carbon atom on the straight chain may be substituted by one OR² group (R² has the same meaning as defined above);
X represents a chlorine atom, a bromine atom, a hydrogen atom or a cyano group;
Y represents any one of the following (1) to (13):
   (1) CO₂R⁵ (R⁵ has the same meaning as defined above),
   (2) a cyano group, (3) OR⁶ (R⁶ represents a hydrogen atom, a C₁₋₄ alkyl group or a phenyl group), (4) a thienyl group,
   (5) a pyridyl group, 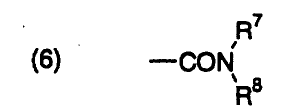
wherein R⁷ and R⁸ each independently represents a hydrogen atom, a C₁₋₄ alkyl group, a C₃₋₈ cycloalkyl group, a phenyl group, a thiazolyl group or a thiadiazolyl group; or R⁷ and R⁸ are combined together to form a straight or branched C₂₋₈ alkylene which may be substituted by a C₁₋₃ alkyl group or a phenyl group or to form a morpholine ring with the nitrogen atom; 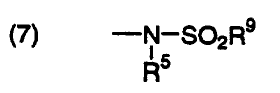 wherein R⁵ has the same meaning as defined above; and R⁹ represents a phenyl group which may be substituted by a hydrogen atom, a C₁₋₄ alkyl group or a halogen atom; 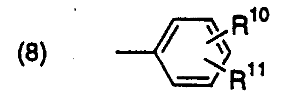 wherein R¹⁰ and R¹¹ each independently represents a hydrogen atom, a halogen atom, a C₁₋₄ alkyl group, a C₁₋₄ acylamino group, OR⁵ (R⁵ has the same meaning as defined above), NHSO₂R⁹ (R⁹ has the same meaning as defined above) or S(O)ₘ-R¹² (where m is an integer of 0 to 2 and R¹² represents a C₁₋₄ alkyl group); 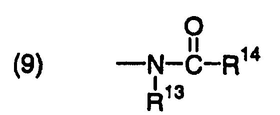 wherein R¹³ represents a hydrogen atom; R¹⁴ represents a phenyl group; or R¹³ and R¹⁴ are combined together to form C₂₋₈ alkylene which may be substituted by a straight C₁₋₃ alkyl group; 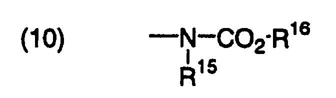 wherein R¹⁵ represents a hydrogen atom or a C₁₋₄ alkyl group; R¹⁶ represents a C₁₋₄ alkyl group; or R¹⁵ and R¹⁶ are combined together to form C₂₋₈ alkylene which may be substituted by a straight C₁₋₃ alkyl group; 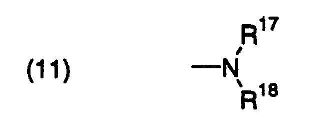 wherein R¹⁷ and R¹⁸ each independently represents a C₁₋₄ alkyl group; or R¹⁷ and R¹⁸ are combined together to form C₂₋₈ alkylene which may be substituted by a straight C₁₋₃ alkyl group; 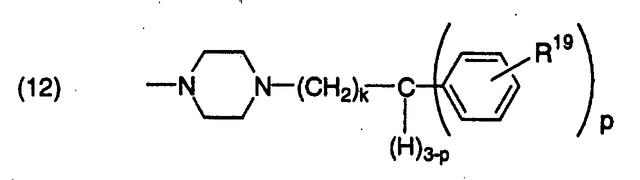 wherein p is 1 or 2, k is an integer of 0 to 3 and R¹⁹ represents a hydrogen atom or a halogen atom or

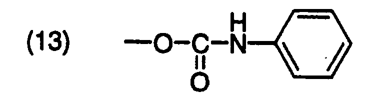

Ar represents any one of the following (1) to (5): 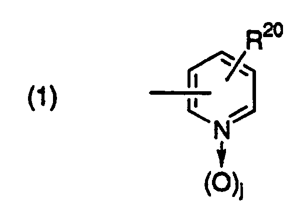 wherein j is 0 or 1, R²⁰ represents a hydrogen atom, a halogen atom or OR¹² (R¹² has the same meaning as defined above), 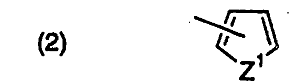 wherein Z¹ represents an oxygen atom or a sulfur atom, 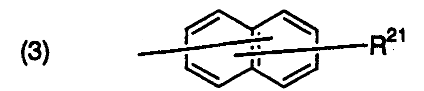 wherein R²¹ represents a hydrogen atom or OR⁵ (R⁵ has the same meaning as defined above), 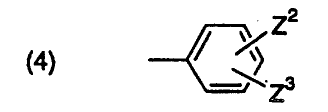 wherein Z² and Z³ each independently represents a hydrogen atom, a halogen atom, a C₁₋₄ alkyl group, OR²² (R²² represents a hydrogen atom or a C₁₋₈ alkyl group), O-A¹-Y¹ (A¹ represents a straight or branched C₁₋₈ alkylene and Y¹ represents a C₁₋₄ alkyl group or a phenyl group which may be substituted by a halogen atom), CO₂R⁵ (R⁵ has the same meaning as defined above) or 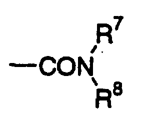 wherein R⁷ and R⁸ have the same meanings as defined above or 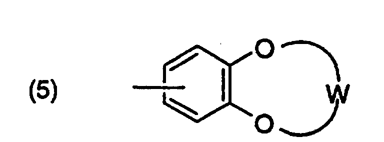 wherein W forms C₁₋₈ alkylene which may be substituted by a straight C₁₋₃ alkyl group,
or a pharmaceutically acceptable salt thereof as an active ingredient.

The pyridazinone compound (I) according to the present invention exhibits a potent cavernous body relaxation action and intracavernous pressure elevating action, and can be used as a remedial agent for erectile dysfunction.

Pyridazinone compound (I) is effective for the treatment of erectile dysfunction as a result of having both PDE III inhibitory activity and PDE V inhibitory activity, and demonstrates considerably improved efficacy as compared with therapeutic agents of the prior art.

Moreover, it is a superior remedial agent for erectile dysfunction by being able to reduce adverse side effects caused by excessive blood pressure lowering effects attributable to PDE V inhibitors, as well as reduce effects on the heart as observed with PDE III inhibitors.

### Brief description of the drawings

Fig. 1 is a diagram showing the effects of Compound A on intracavernous pressure, duration and AUC in anesthetized dogs.
Fig. 2 is a diagram showing the effects resulting from concomitant use of milrinone and zaprinast on intracavernous pressure, duration and AUC in anesthetized dogs.

### Best mode for carrying out the invention

In the following, the substituents in the compound relating to the present invention of the above formula (I), i.e., R¹, R², R³, R⁴, X, Y, A and Ar are explained.

Specific examples of R¹ may include a hydrogen atom; a methyl group, an ethyl group, a n-propyl group, an i-propyl group, a n-butyl group, an i-butyl group, a sec-butyl group, a t-butyl group; a 2-propenyl group, a 2-methyl-2-propenyl group; a carboxymethyl group, a 2-carboxyethyl group, a 3-carboxypropyl group, a 4-carboxybutyl group, a methoxycarbonylmethyl group, a 2-methoxycarbonylethyl group, a 3-methoxycarbonylpropyl group, a 4-methoxycarbonylbutyl group, an ethoxycarbonylmethyl group, a 2-ethoxycarbonylethyl group, a 3-ethoxycarbonylpropyl group, a 4-ethoxycarbonylbutyl group, a n-propoxycarbonyl-methyl group, an i-propoxycarbonylmethyl group, a 2-n-propoxycarbonylethyl group, a 2-i-propoxycarbonylethyl group, a 3-n-propoxycarbonylpropyl group, a 3-i-propoxycarbonylpropyl group, a 4-n-propoxycarbonylbutyl group, a 4-i-propoxycarbonylbutyl group, a n-butoxycarbonylmethyl group, an i-butoxycarbonylmethyl group, a sec-butoxycarbonylmethyl group, a t-butoxycarbonylmethyl group, a 2-n-butoxycarbonylmethyl group, a 2-i-butoxycarbonylethyl group, a 2-sec-butoxycarbonylethyl group, a 2-t-butoxycarbonylethyl group, a 3-n-butoxycarbonylpropyl group, a 3-i-butoxycarbonylpropyl group, a 3-sec-butoxycarbonylpropyl group, a 3-i-butoxycarbonylpropyl group, a 4-n-butoxycarbonylbutyl group, a 4-i-butoxycarbonylbutyl group, a 4-sec-butoxycarbonylbutyl group, a 4-t-butoxycarbonylbutyl group, etc., preferably a hydrogen atom, an ethyl group and an i-propyl group, more preferably a hydrogen atom.

R² may include a hydrogen atom, a methyl group, an ethyl group, a n-propyl group, an i-propyl group, a n-butyl group, an i-butyl group, a sec-butyl group and a t-butyl group, more preferably a hydrogen atom and a methyl group.

Specific examples of R³ and R⁴ may include a hydrogen atom, a methyl group, an ethyl group, a n-propyl group and an i-propyl group, preferably a hydrogen atom.

A represents a single bond or a straight or branched C₁₋₁₁ alkylene in which one carbon atom on a straight chain may be substituted by one hydroxyl group or a C₁₋₄ alkoxy group, and specifically includes a single bond; methylene, hydroxymethylene, methoxymethylene, ethoxymethylene, propoxymethylene, butoxymethylene, ethylene, 1hydroxyethylene, 2-hydroxyethylene, 1-methoxyethylene, 2-methoxyethylene, 1-ethoxyethylene, 2-ethoxyethylene, 1-propoxyethylene, 2-propoxyethylene, 1-butoxyethylene, 2-butoxyethylene, propylene, 1-hydroxypropylene, 2-hydroxypropylene, 3-hydroxypropylene, 1-methoxypropylene, 2-methoxypropylene, 3-methoxypropylene, 1-ethoxypropylene, 2-ethoxypropylene, 3-ethoxypropylene, 1-propoxypropylene, 2-propoxypropylene, 3-propoxypropylene, 1-butoxypropylene, 2-butoxypropylene, 3-butoxypropylene, butylene, 1-hydroxybutylene, 2-hydroxybutylene, 3-hydroxybutylene, 4-hydroxybutylene, 1-methoxybutylene, 2-methoxybutylene, 3-methoxybutylene, 4-methoxybutylene, 1-ethoxybutylene, 2-ethoxybutylene, 3-ethoxybutylene, 4-ethoxybutylene, 1-propoxybutylene, 2-propoxybutylene, 3-propoxybutylene, 4-propoxybutylene, 1-butoxybutylene, 2-butoxybutylene, 3-butoxybutylene, 4-butoxybutylene, pentylene, 1-hydroxypentylene, 2-hydroxypentylene, 3-hydroxypentylene, 4-hydroxypentylene, 5-hydroxypentylene, 6-hydroxyhexylene, heptylene, 7-hydroxyheptylene, octylene, 8-hydroxyoctylene, nonylene, 9-hydroxynonylene, decanylene, 10-hydroxydecanylene, undecanylene, 11-hydroxyundecanylene, hydroxymethylmethylene, ethyl-hydroxymethylene, hydroxy-propylmethylene, methoxy-methylmethylene, methylmethylene, ethylmethylene, propylmethylene, dimethylmethylene, diethylmethylene, dipropylmethylene, ethyl-methylmethylene, methyl-propylmethylene, 1-hydroxy-1-methylethylene, 1-ethyl-1-hydroxyethylene, 1-hydroxy-1-propylethylene, 1-methoxy-1-methylethylene, 1-hydroxyl-2-methylethylene, 2-ethyl-1-hydroxyethylene, 1-hydroxy-2,2-dimethylethylene, 2,2-diethyl-1-hydroxyethylene, 1-methoxy-2,2-dimethylethylene, 2-hydroxy-1-methylethylene, 1-ethyl-2-hydroxyethylene, 2-hydroxy-1,1-dimethylethylene, 1,1-diethyl-2-hydroxyethylene, 2-methoxy-1,1-dimethylethylene, 1-methylethylene, 1,1-dimethylethylene, 1,1-diethylethylene, 2-methylethylene, 2,2-dimethylethylene, 1,2-dimethylethylene, 2,2-diethylethylene, 1,1,2,2-tetramethylethylene, 1-hydroxy-1-methylpropylene, 1-methoxy-1-methylpropylene, 1-hydroxy-2-methylpropylene, 1-methoxy-2-methylpropylene, 1-hydroxy-3-methylpropylene, 1-methoxy-3-methylpropylene, 1-hydroxy-2,2-dimethylpropylene, 1-methoxy-2,2-dimethylpropylene, 2,2-diethyl-1-hydroxypropylene, 1-hydroxy-3,3-dimethylpropylene, 1-methoxy-3,3-dimethylpropylene, 3,3-diethyl-1-hydroxypropylene, 1-hydroxy-2,2,3,3-tetramethylpropylene, 2-hydroxy-1-methylpropylene, 2-methoxy-1-methylpropylene, 2-hydroxy-2-methylpropylene, 2-methoxy-2-methylpropylene, 2-hydroxy-3-methylpropylene, 2-methoxy-3-methylpropylene, 2-hydroxy-1,1-dimethylpropylene, 2-methoxy-1,1-dimethylpropylene, 1,1-diethyl-2-hydroxypropylene, 2-hydroxy-3,3-dimethylpropylene, 2-methoxy-3,3-dimethylpropylene, 3,3-diethyl-2-hydroxypropylene, 2-hydroxy-1,1,3,3-tetramethylpropylene, 3-hydroxy-1-methylpropylene, 3-methoxy-1-methylpropylene, 3-hydroxy-2-methylpropylene, 3-methoxy-2-methylpropylene, 3-hydroxy-3-methylpropylene, 3-methoxy-3-methylpropylene, 3-hydroxy-1,1-dimethylpropylene, 3-methoxy-1,1-dimethylpropylene, 1,1-diethyl-3-hydroxypropylene, 3-hydroxy-2,2-dimethylpropylene, 3-methoxy-2,2-dimethylpropylene, 2,2-diethyl-3-hydroxypropylene, 3-hydroxy-3-methylpropylene, 3-hydroxy-1,1,2,2-tetramethylpropylene, 1-methylpropylene, 1-ethylpropylene, 1-propylpropylene, 2-methylpropylene, 2-ethylpropylene, 2-propylpropylene, 3-methylpropylene, 3-ethylpropylene, 3-propylpropylene, 1,1-dimethylpropylene, 2,2-dimethylpropylene, 3,3-dimethylpropylene, 1,1-diethylpropylene, 2,2-diethylpropylene, 3,3-diethylpropylene, 1,1-dipropylpropylene, 2,2-dipropylpropylene, 3,3-dipropylpropylene, 2,2-dimethyl-1-hydroxybutylene, 2,2-dimethyl-1-methoxybutylene, 3,3-dimethyl-1-hydroxybutylene, 3,3-dimethyl-1-methoxybutylene, 4,4-dimethyl-1-hydroxybutylene, 4,4-dimethyl-1-methoxybutylene, 1,1-dimethyl-2-hydroxybutylene, 1,1-dimethyl-2-methoxybutylene, 3,3-dimethyl-2-hydroxybutylene, 3,3-dimethyl-2-methoxybutylene, 4,4-dimethyl-2-hydroxybutylene, 4,4-dimethyl-2-methoxybutylene, 1,1-dimethyl-3-hydroxybutylene, 1,1-dimethyl-3-methoxybutylene, 2,2-dimethyl-3-hydroxybutylene, 2,2-dimethyl-3-methoxybutylene, 4,4-dimethyl-3-hydroxybutylene, 4,4-dimethyl-3-methoxybutylene, 1,1-dimethyl-4-hydroxybutylene, 1,1-dimethyl-4-methoxybutylene, 2,2-dimethyl-4-hydroxybutylene, 2,2-dimethyl-4-methoxybutylene, 3,3-dimethyl-4-hydroxybutylene, 3,3-dimethyl-4-methoxybutylene, 5,5-dimethylpentylene, 6,6-dimethylhexylene, 7,7-dimethylheptylene, 8,8-dimethyloctylene, etc., preferably C₁₋₅ alkylene and ω-dialkyl- or ω-hydroxy-alkylene in the carbon chain thereof.

Specific examples of Y may include a carboxyl group, a methoxycarbonyl group, an ethoxycarbonyl group, a n-propoxycarbonyl group, an i-propoxycarbonyl group, a n-butoxycarbonyl group, an i-butoxycarbonyl group, a secbutoxycarbonyl group, a t-butoxycarbonyl group; a 2-thienyl group, a 3-thienyl group, a 2-pyridyl group, a 3-pyridyl group, a 4-pyridyl group; a cyano group; a hydroxyl group, a methoxy group, an ethoxy group, a n-propoxy group, an i-propoxy group, a n-butoxy group, an i-butoxy group, a sec-butoxy group, a t-butoxy group; a phenoxy group; a carbamoyl group, a N-methylaminocarbonyl group, a N-ethylaminocarbonyl group, a N-n-propylaminocarbonyl group, a N-i-propylaminocarbonyl group, a N-n-butylaminocarbonyl group, a N-i-butylaminocarbonyl group, a N-sec-butylaminocarbonyl group, a N-t-butylaminocarbonyl group; a N-cyclopropylaminocarbonyl group, a N-cyclobutylaminocarbonyl group, a N-cyclopentylaminocarbonyl group, a N-cyclohexylaminocarbonyl group, a N-cycloheptylaminocarbonyl group, a N-cyclooctylaminocarbonyl group; a N-phenylaminocarbonyl group, a N-2-thiazolylaminocarbonyl group, a N-4-thiazolylaminocarbonyl group, a N-5-thiazolylaminocarbonyl group, a N-2-thiadiazolylaminocarbonyl group, a N-5-thiadiazolylaminocarbonyl group, a 1-aziridinocarbonyl group, 1-azetidinocarbonyl group, a 1-pyrrolidinocarbonyl group, a 1piperidinocarbonyl group, a 1-homopiperidinocarbonyl group, 1-(2,5-dimethyl)pyrrolidinocarbonyl group, a 1-(2,6-dimethyl)piperidinocarbonyl group, 1-(3-phenyl)pyrrolidinocarbonyl group, 1-(4-phenyl)piperidinocarbonyl group, 1-morpholinocarbonyl group; a N-methyl-sulfonylamino group, a N-ethyl-sulfonylamino group, a N-n-propyl-sulfonylamino group, a N-i-propyl-sulfonylamino group, a N-n-butylsulfonylamino group, a N-i-butyl-sulfonylamino group, a N-sec-butyl-sulfonylamino group, a N-t-butyl-sulfonylamino group, a N-phenyl-sulfonylamino group, a phenylsulfonylamino group in which an ortho-position, a meta-position or a para-position on the benzene ring is substituted by a methyl group, an ethyl group, a n-propyl group, an i-propyl group, a n-butyl group, an i-butyl group, a sec-butyl group, a t-butyl group, a fluorine atom, a chlorine atom, a bromine atom or an iodine atom; a phenyl group or a mono-or di-substituted phenyl group in which an arbitrary position(s) on the benzene ring is/are each independently substituted by 1 or 2 substituents selected from a methyl group, an ethyl group, a n-propyl group, an i-propyl group, a n-butyl group, an i-butyl group, a sec-butyl group, a t-butyl group, a fluorine atom, a chlorine atom, a bromine atom, an iodine atom, a formylamino group, an acetylamino group, a propionylamino group, a butyrylamino group, a methylsulfonylamino group, an ethylsulfonylamino group, a n-propylsulfonylamino group, an i-propylsulfonylamino group, a n-butylsulfonylamino group, an i-butylsulfonylamino group, a sec-butylsulfonylamino group, a t-butylsulfonylamino group, a phenylsulfonylamino group, a hydroxyl group, a methoxy group, an ethoxy group, a n-propoxy group, an i-propoxy group, a n-butoxy group, an i-butoxy group, a sec-butoxy group, a t-butoxy group, a methylthio group, an ethylthio group, a n-propylthio group, an i-propylthio group, a n-butylthio group, an i-butylthio group, a sec-butylthio group, a t-butylthio group, a methylsulfoxy group, an ethylsulfoxy group, a n-propylsulfoxy group, an i-propylsulfoxy group, a n-butylsulfoxy group, an i-butylsulfoxy group, a sec-butylsulfoxy group, a t-butylsulfoxy group, a methylsulfonyl group, an ethylsulfonyl group, a n-propylsulfonyl group, an i-propylsulfonyl group, a n-butylsulfonyl group, an i-butylsulfonyl group, a sec-butylsulfonyl group and a t-butylsulfonyl group; a benzoylamino group, a 1-(2-oxo)azetidinyl group, a 1-(2-oxo)pyrrolidinyl group, a 1-(2-oxo)piperidinyl group, a 1-(2-oxo)homopiperidinyl group, a 1-(2-oxo-3,3-dimethyl)-pyrrolidinyl group, a 1-(2-oxo-4,4-dimethyl)pyrrolidinyl group, a 1-(2-oxo-5,5-dimethyl)pyrrolidinyl group; a N-methoxycarbonylamino group, a N-ethoxycarbonylamino group, a N-n-propoxycarbonylamino group, a N-i-propoxycarbonylamino group, a N-n-butoxycarbonylamino group, a N-i-butoxycarbonylamino group, a N-sec-butoxycarbonylamino group, a N-t-butoxycarbonylamino group, a 3-(2-oxo)oxazolidinyl group, a 3-(2-oxo-5,5-dimethyl)oxazolidinyl group, a 3-(2-oxo-4,4-diethyl)oxazolidinyl group, a 3-(2-oxo-5,5-diethyl)oxazolidinyl group; a N,N-dialkyl substituted amino group in which a methyl group, an ethyl group, a n-propyl group, an i-propyl group, a n-butyl group, an i-butyl group, a sec-butyl group and a t-butyl group are substituted in arbitrary combination; a 1-azetidino group, a 1-pyrrolidino group, a 1-piperidino group, a 1-(2,5-dimethyl)pyrrolidino group, a 1-(3,4-dimethyl)pyrrolidino group, a 1-(4,4-dimethyl)piperidino group, a 1-(4-benzyl)-piperazino group, a 1-(4-diphenylmethyl)piperazino group; a 1-(4-substituted benzyl)piperazinyl group or a 1-(4-di-substituted phenylmethyl)piperazinyl group in which an ortho-position, a meta-position or a para-position on the benzene ring is/are substituted by a fluorine atom, a chlorine atom, a bromine atom or an iodine atom; a phenylaminocarbonyloxy group or a N,N-di-substituted aminocarbonyl group in which the N-substituted aminocarbonyl groups explained above are substituted by a chain state or cyclic alkyl group, a phenyl group, a thiazolyl group or a thiadiazolyl group in an arbitrary combination; or a N-alkylalkylsulfonylamino group, a N-alkyl-phenylsulfonylamino group or a N-alkyl-alkoxycarbonylamino group in which the nitrogen atom of the alkylsulfonylamino group, the phenylsulfonylamino group or the alkoxycarbonylamino group explained above is further substituted by a straight or branched C₁₋₄ alkyl group.

Specific examples of X may include a hydrogen atom, a chlorine atom, a bromine atom and a cyano group, preferably a chlorine atom, a bromine atom and a cyano group.

Specific examples of Ar may include a 2-pyridyl group, a 3-pyridyl group, a 4-pyridyl group, a substituted 2-pyridyl group, 3-pyridyl group or 4-pyridyl group in which 2-, 3-, 4-, 5- or 6-position on the pyridine ring is substituted by a fluorine atom, a chlorine atom, a bromine atom, an iodine atom, a methoxy group, an ethoxy group, a n-propoxy group, an i-propoxy group, a n-butoxy group, an i-butoxy group, a sec-butoxy group or a t-butoxy group; a group in which the pyridyl group or the substituted pyridyl group explained above is replaced by a N-oxidopyridyl group, a 2-furyl group, a 3-furyl group, a 2-thienyl group, a 3-thienyl group, a 1-naphthyl group or a 2-naphthyl group; a 1-naphthyl group or a 2-naphtyl group in which an arbitrary position on the naphthalene ring is substituted by a hydroxyl group, a methoxy group, an ethoxy group, a n-propoxy group, an i-propoxy group, a n-butoxy group, an i-butoxy group, a sec-butoxy group or a t-butoxy group; and a substituted phenyl group substituted by the following 1 or 2 substituents at an arbitrary position(s) and arbitrary combination. Specific examples of the substituent may include a hydrogen atom, a fluorine atom, a chlorine atom, a'bromine atom, an iodine atom, a methyl group, an ethyl group, a n-propyl group, an i-propyl group, a n-butyl group, an i-butyl group, a sec-butyl group, a t-butyl group, a hydroxyl group, a straight or branched C₁₋₈ alkoxy group; a methylenedioxy group, an ethylenedioxy group or a propylenedioxy group in which two adjacent substituents are combined together; or O-A¹-Y¹ group. Here, A¹ represents a straight or branched C₁₋₈ alkylene and Y¹ represents a phenyl group, a substituted phenyl group in which an ortho-position, a meta-position or a para-position on the benzene ring is substituted by a methyl group, an ethyl group, a n-propyl group, an i-propyl group, a n-butyl group, an i-butyl group, a sec-butyl group, a t-butyl group, a fluorine atom, a chlorine atom, a bromine atom or an iodine atom; a carboxyl group, an alkoxycarbonyl group, a 1-cyclic aminocarbonyl group, a 1-morpholinocarbonyl group explained in the item of Y; a carbamoyl group in which two substituents selected from a hydrogen atom, a straight, branched or cyclic alkyl group, a phenyl group, a thiazolyl group and a thiadiazolyl group are bonded to a nitrogen atom in an arbitrary combination, a N-substituted or N,N-di-substituted aminocarbonyl group.

Among them, preferred groups may include a 3-pyridyl group and a 3-substituted-4-methoxyphenyl type one, but the present invention is not limited to these specific examples.

In the above explanation, n means normal, i means iso, sec means secondary and t means tertiary.

Preferred compounds represented by the formula (I) to be used in the present invention may include the following compounds.
(1) A remedial agent for erectile dysfunction represented by the formula (I) in which R¹ is a hydrogen atom, X is a chlorine atom or a bromine atom and R³ and R⁴ are both hydrogen atoms.
(2) The remedial agent for,erectile dysfunction described in the above (1) in which Ar is a pyridyl group, a N-oxypyridyl group or a phenyl group represented by the formula: 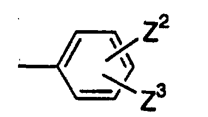 wherein Z² and Z³ each independently represents a hydrogen atom, a halogen atom, a C₁₋₄ alkyl group or OR²² (R²² represents a hydrogen atom or a C₁₋₈ alkyl group).
(3) The remedial agent for erectile dysfunction described in any one of the above (1) to (3) in which Y is a carbamoyl group represented by the formula: 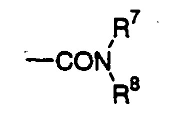 wherein R⁷ and R⁸ each independently represents a hydrogen atom, a C₁₋₄ alkyl group, a C₃₋₈ cycloalkyl group or a phenyl group, or R⁷ and R⁸ are combined together to form C₂₋₈ alkylene which may be substituted by a C₁₋₃ alkyl group or a phenyl group or to form a morpholine ring with a nitrogen atom; or a phenyl group represented by the formula: 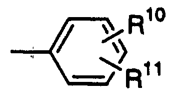 wherein R¹⁰ and R¹¹ each independently represents a hydrogen atom, a halogen atom, a C₁₋₄ alkyl group, a C₁₋₄ acylamino group, OR⁵ (R⁵ represents a hydrogen atom or a C₁₋₄ alkyl group), NHSO₂R⁹ (R⁹ represents a phenyl group which may be substituted by a hydrogen atom, a C₁₋₄ alkyl group or a halogen atom) or S(O)ₘ-R¹² (m is an integer of 0 to 2 and R¹² represents a C₁₋₄ alkyl group).
(4) The remedial agent for erectile dysfunction described in the above (1) or (2) in which Ar is a pyridyl group, a N-oxypyridyl group or a phenyl group which may be substituted by OR²² (R²² represents a C₁₋₄ alkyl group).
(5) The remedial agent for erectile dysfunction described in any one of the above (1) to (4) in which A is a straight or branched C₁₋₈ alkylene in which a carbon atom on the straight chain may be substituted by one hydroxyl group (-OH).
(6) The remedial agent for erectile dysfunction described in any one of the above (1) to (5) in which Y is a phenyl group represented by the formula: 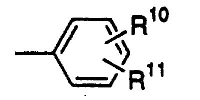 wherein R¹⁰ and R¹¹ each independently represents a hydrogen atom, a halogen atom, a C₁₋₄ alkyl group, a C₁₋₄ acylamino group, OR⁵ (R⁵ represents a hydrogen atom or a C₁₋₄ alkyl group), NHSO₂R⁹ (R⁹ represents a phenyl group which may be substituted by a hydrogen atom, a C₁₋₄ alkyl group or a halogen atom) or S(O)ₘ-R¹² (m is an integer of 0 to 2 and R¹² represents a C₁₋₄ alkyl group).
(7) The remedial agent for erectile dysfunction described in the above (6) in which Y is a phenyl group which may be substituted by a halogen atom.
(8) The remedial agent for erectile dysfunction described in any one of the above (4) to (7) in which Ar is a 3-pyridyl group.
(9) The remedial agent for erectile dysfunction described in the formula (I) wherein the compound represented by the formula (I) is 4-bromo-6-[3-(4-chlorophenyl)propoxy]-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.

Stereoisomers and optical isomers are also included in the pyridazinone compound (I) and its pharmacologically acceptable salt of the present invention.

The pyridazinone compound (I) and its pharmacologically acceptable salt are known compounds, and these compounds can be produced according to the methods disclosed in, for example, Japanese Unexamined Patent Publication No. Sho 63-301870 (US Patent No. 4,978,665, European Patent No. 275997B), Japanese Patent Publication No. Hei 7-107055 (US Patent No. 5,314,883, European Patent No. 482208B) and Japanese Unexamined Patent Publication No. Hei 7-252237 (US Patent No. 5,750,523, European Patent No. 742211A).

The pyridazinone derivative (I) and its pharmacologically acceptable salt of the present invention have superior treatment effects on erectile dysfunction in mammals such as men, dogs, cows, horses, rabbits, mice and rats.

The dose of the pyridazinone derivative (I) or its pharmacologically acceptable salt according to the present invention may be suitably selected according to the age and body weight of the patient and the degree of symptoms, and the human adult dose is normally 0.001 mg to 5 g per day, and preferably 0.005 to 1000 mg per day administered to him from once to several times by dividing per day.

Examples of administration forms of the pyridazinone derivative (I) or a pharmacologically acceptable salt thereof according to the present invention include parenteral administration forms such as injections (subcutaneous, intravenous, intramuscular and intraperitoneal injections), ointments, suppositories and aerosols, as well as oral administration forms such as tablets, capsules, granules, pills, powders, troches, chewable preparations, syrups, liquids, emulsions and suspensions. Oral administration is preferred.

The compound according to the present invention is formulated for administration by routine means for preparation.

Tablets, capsules, granules and pills for oral administration can be prepared by routine means using powders, troches, chewable preparations, a vehicle (e.g., saccharose, lactose, glucose, starch or mannitol), binder (e.g., syrup, gum arabic, gelatin, sorbitol, tragacanth, methyl cellulose or polyvinyl pyrrolidone), disintegrating agent (e.g., starch, carboxymethyl cellulose or its calcium salt, microcrystalline cellulose or polyethylene glycol), lubricant (e.g., talc, magnesium stearate, calcium stearate or silica), smoothing agent (e.g., sodium laurate or glycerin) and so forth.

In addition, injections, aerosols, syrups, liquids, emulsions, suspensions and so forth are prepared by routine means using a solvent (e.g., water, ethyl alcohol, isopropyl alcohol, propylene glycol, 1,3-butylene glycol or polyethylene glycol) of the active ingredient, surfactant (e.g., sorbitan fatty acid ester, polyoxyethylene sorbitan fatty acid ester, polyoxyethylene fatty acid ester, polyoxyethylene ether of hydrogenated castor oil or lecithin), suspending agent (e.g., carboxymethyl sodium salt, cellulose derivatives such as methyl cellulose, or natural rubbers such as tragacanth or gum arabic), preservative (e.g., an ester of paraoxybenzoic acid, benzalkonium chloride or sorbates) and so forth. Suppositories are produced by routine methods using, for example, cacao butter, polyethylene glycol, lanolin, fatty acid triglycerides, coconut oil and so forth.

White petrolatum, liquid paraffin, higher alcohols, macrogoll ointment, hydrophilic ointment, aqueous gel base and so forth are used for ointments used as preparations that are absorbed through the skin.

### Experimental examples and Examples

The present invention will be described in detail by way of experimental examples and examples, but the present invention is not limited in any way to these examples.

Compound A (4-bromo-6-[3-(4-chlorophenyl)propoxy]-5-(3-pyridylmethylamino)-3(2H)-pyridazinone hydrochloride) and Compound B (4-bromo-6-[3-(4-chlorophenyl)-3-hydroxypropoxy]-5-(3-pyridylmethylamino)-3(2H)-pyridazinone hydrochloride) produced in accordance with the conventional manner were used as reagents. Commercially available products were used for the other reagents.

### Experimental example 1

### Activity on intracavernous pressure by intracavernous injection to anesthetized dogs

Male beagle dogs having body weights of about 10 kg were anesthetized by intravenous administration of sodium pentobarbital at 35 mg/kg followed by immobilizing the animals in the dorsal position on a heating mat (SMS-2000J, Medical System Inc.) and inserting a. cannula into the trachea. A 23G winged needle for measuring internal pressure was inserted into the right corpus cavernosum, and intracavernous pressure was measured with a blood pressure measurement amplifier (AP-641G, Nihon Koden) by means of a pressure transducer (Statham P-50, Gould Co.). Moreover, a 23G winged needle for administration of drug was inserted into the right corpus cavernosum of the base followed by intracavernous injection of sodium nitroprusside (SNP, 10⁻⁴ or 10⁻³ mol/L, 0.5 ml) followed by administration of drug at the stage an increase in intracavernous pressure was confirmed. It should be noted that Compound A and milrinone were prepared with dimethyl sulfoxide-ethanol-physiological saline (1:2:30). Zaprinast was used after dissolving in 1 mol/L aqueous sodium hydroxide solution and adjusting the pH to the vicinity of 9.5 with 1 mol/L hydrochloric acid.

The activity of Compound A was assessed directly by administering at a volume of 0.5 ml starting from the lowest dose level at 0.243, 0.729, 2.43, 7.29, 24.3 and 72.9 µg/body (10⁻⁶, 3 × 10⁻⁶, 10⁻⁵, 3 × 10⁻⁵, 10⁻⁴ and 3 × 10⁻⁴ mol/L).

The effect of milrinone and zaprinast was assessed by concomitant administration of milrinone at 106 µg/body (10⁻³ mol/L) and zaprinast at 136 µg/body (10⁻³ mol/L) using a dosing volume of 0.5 ml each.

Intracavernous pressure was input into a polygraph (RM-6000, Nihon Koden), and together with continuously recording on a thermal pen recorder, was recorded and sampled on an external storage device (hard disk) of a computer (Macintosh Performa 5260, Apple) by means of a circulation kinetics analysis system (MP100WS, BIOPAC System) using circulation kinetics analysis software (MP/VAS 3 Ver. 1.0 beta 4.2, Physio-Tech Co., Ltd.). Maximum rate of change, duration and area under the curve (AUC) of intracavernous pressure were calculated using the circulation kinetics analysis software based on the resulting data.

The resulting values were expressed as the mean ± standard error (S.E.) and subjected to the statistical processing indicated below. The effect of Compound A was tested according to Dunnett's method using the solvent group as the control. The concomitant effect of milrinone and zaprinast was analyzed by two-way analysis of variance. A level of P<0.05 was considered to be statistically significant in all tests.

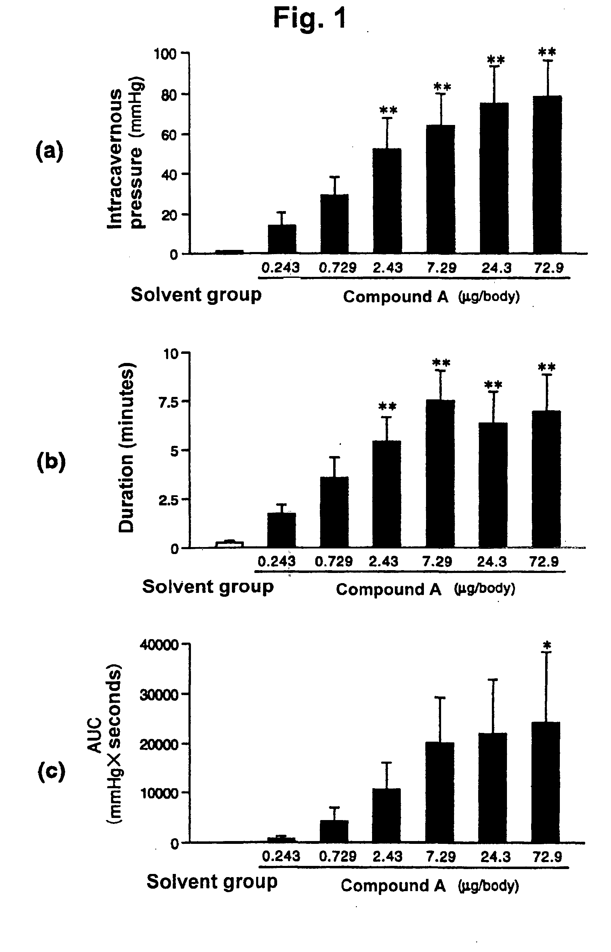

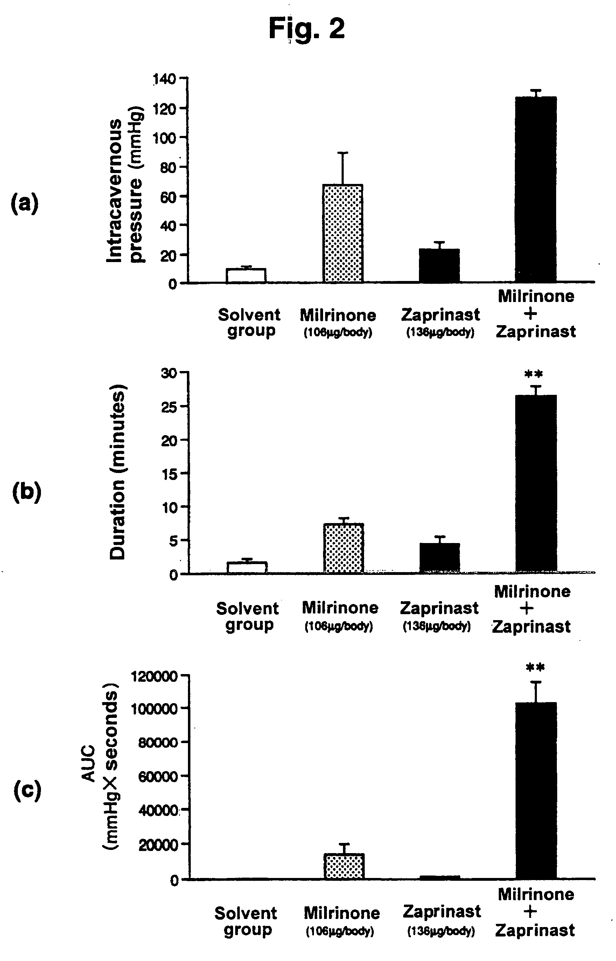

The results are shown in Fig. 1 and Fig. 2.
i) Direct effect on intracavernous pressure
   Compound A increased intracavernous pressure, nearly dose-dependently from 0.243 to 72.9 µg/body (10⁻⁶ to 3 × 10⁻⁴ mol/L). Significant effects were observed with respect to maximum rate of change and duration at 2.43 µg/body (10⁻⁵ mol/L) and above. As a result, similar effects were observed for AUC as well (Fig. 1).
   It should be noted that in Fig. 1, asterisks (*) indicate the presence of a significant difference at p<0.05 as a result of performing Dunnett's test using the solvent group as the control.
   In addition, in Fig. 1, double asterisks (**) indicate the presence of a significant difference at p<0.01 as a result of performing Dunnett's test using the solvent group as the control.
ii) Effect of concomitant administration of milrinone and zaprinast
   Concomitant administration of milrinone at 106 µg/body (10⁻³ mol/L) and zaprinast at 136 µg/body (10⁻³ mol/L) remarkably increased intracavernous pressure as compared with administration of either drug alone, and significant synergistic effects were observed with respect to duration and AUC (Fig. 2).
   Be noted that in Fig. 2, double asterisks (**) indicate the presence of a significant difference at p<0.01 as a result of administering milrinone or zaprinast alone and performing two-way analysis of variance.

### Experimental example 2

### Relaxation on norepinephrine-induced contraction in isolated corpus cavernosum of rabbit

Rabbits were sacrificed by venesection following severing of the carotid artery under sodium pentobarbital anesthesia. The penis was immediately isolated and transferred into Krebs-Henseleit solution (composition (mmol/L): NaCl 118.4, KCl 4.7, CaCl₂ 2.5, MgSO₄ 1.2, KH₂PO₄ 1.2, NaHCO₃ 25.0, glucose 11.1), which was adequately aerated with a 95% O₂/5% CO₂. After cutting away the connective tissue, the penis was severed longitudinally into two sections so that half of the urethra was present in each section by using an extension of the midline as the boundary. Each preparation was suspended from an FD pickup (TB-611T, Nihon Koden) at a resting tension of 0.5 g (about 5 mN) for the load in a nutrient solution consisting of 20 ml of organ bath maintained at 37°C. Contraction was recorded as the isometric contraction on a thermal pen writing recorder (WT-685G, Nihon Koden) following amplification by a carrier amplifier (AP-621G, Nihon Koden). After replacing the nutrient solution twice every 20 minutes and allowing to equilibrate, reactivity was confirmed for each preparation by observing the contraction to 1 µmol/L of norepinephrine. After contraction stabilized, the preparation was washed by replacing the nutrient solution, after which the solution was additionally replaced twice every 20 minutes and acceptable to equilibrate.

To begin with, each preparation was contracted by norepinephrine (final concentration: 1 µmol/L). The preparation for which stable tension was obtained at this time was used in the experiment. Each drug was administered cumulatively in 10-fold ratios followed by observation of the reaction. The maximum relaxation reaction was determined by application of papaverine hydrochloride (final concentration: 100 µmol/L).

The inhibition rate at each concentration was calculated by taking the tension during addition of norepinephrine to represent an inhibition rate of 0%, and the tension during the maximum relaxation reaction induced by addition of papaverine hydrochloride to represent an inhibition rate of 100%, followed by calculation of the concentration value at which the inhibition rate was 50% (IC₅₀).

Those results are shown in Table 1 and Table 2.
i) Direct relaxation on norepinephrine-induced contraction in isolated corpus cavernosum of rabbit
All of the drugs demonstrated dose-dependent relaxation action. The IC₅₀ values of each compound were as shown in the table below. The action of Compound A was the most potent.

**Table 1**

| | IC₅₀ (µmol/L) |
|---|---|
| Compound A | 0.33 |
| Compound B | 1.59 |
| Milrinone | 5.8 |
| Zaprinast | 5.5 |

ii) Relaxation on norepinephrine-induced contraction in isolated corpus cavernosum of rabbit in the presence of SNP and N-omega-nitro-L-arginine methyl ester (L-NAME)
The action of Compound A was enhanced about two-fold by pre-treatment with SNP (1 nmol/L), and decreased by about one-half by pre-treatment with L-NAME (1 mmol/L). On the other hand, the action of zaprinast was attenuated to 1/18 or less by pre-treatment with L-NAME (1 mmol/L).

**Table 2**

| | IC₅₀ (µmol/L) |
|---|---|
| Compound A | 0.33 |
| Compound A + SNP | 0.15 |
| Compound A + L-NAME | 0.77 |
| Zaprinast | 5.5 |
| Zaprinast + L-NAME | >100 |

### Experimental example 3

### Relaxation on norepinephrine-induced contraction in isolated thoracic aorta of rabbit

The procedure was performed in the same manner as Experimental Example 2 with the exception of isolating the thoracic aorta and preparing in the form of a spinal strip, and using 1 g (about 10 mN) of resting tension for the load.

The results are shown in Table 3 and Table 4.
i) Direct relaxation on norepinephrine-induced contraction in isolated thoracic aorta of rabbit
Compound A exhibited dose-dependent relaxation action, and its IC₅₀ value was 2.65 µmol/L. When compared with the action on the corpus cavernosum in Experimental Example 2, the action on the cavernous body was about 8 times more potent than that on the aorta.

**Table 3**

| | IC₅₀ (µmol/L) |
|---|---|
| Aorta | 2.65 |
| Cavernous body | 0.33 |

ii) Relaxation on norepinephrine-induced contraction in the isolated thoracic aorta of rabbit in the presence of SNP
The action of Compound A was completely unaffected by pre-treatment with SNP (1 nmol/L), and results were obtained that differed from the action on the corpus cavernosum in Experimental example 2.

**Table 4**

| | IC₅₀ (µmol/L) | |
|---|---|---|
| | Corpus cavernosum | Aorta |
| Compound A | 0.33 | 2.65 |
| Compound A + SNP | 0.15 | 2.71 |

### Experimental example 4

### Action on isolated cardiac muscle of guinea-pig

After sacrificing male Hartley guinea-pigs by venesection, the heart was isolated and transferred to Krebs-Henseleit solution was adequately aerated with a 95% O₂/5% CO₂. After promptly separating the heart into the atria and ventricles, a papillary muscle preparation from the right ventricle and a right atrium preparation were prepared. Each preparation was suspended from an FD pickup (TB-611T, Nihon Koden) at a resting tension of 0.5 g (about 5 mN) for the load in a nutrient solution consisting of organ bath maintained at 31°C.

Automatic contraction of the right atrium amplified by a carrier amplifier (AP-600G, Nihon Koden) followed by recording tension and the number of beats on a recorded by means of an instantaneous heart rate counter (AT600G, Nihon Koden). For the papillary muscle, a rectangular wave electrical stimulus was applied by an electrical stimulator (SEN-3201, Nihon Koden) through bipolar platinum electrodes followed by amplification of the resulting tension by a carrier amplifier (AP-600G, Nihon Koden) and recording on a recorder.

The right atrium preparations were equilibrated by replacing the nutrient solution twice every 20 minutes followed by confirmation of the reactivity of each preparation by observing the number of beats in the presence of 0.1 µmol/L of isoproterenol. After the number of beats had stabilized, the preparations were washed by replacing the nutrient solution and additionally equilibrated by replacing the nutrient solution twice every 20 minutes. Each drug was administered cumulatively followed by observing the reaction. Drug action was evaluated by determining the rate of change caused by the drug by taking the difference between the maximum reaction induced by isoproterenol and that before drug administration to be 100%, and then calculating the EC₃₀ value as the concentration at which drug action was 30% with respect to the number of beats based on this rate of change.

The papillary muscle preparations were equilibrated by replacing the nutrient solution twice every 20 minutes followed by cumulative administration of isoproterenol (final concentration: 0.03 µmol/L) and observation of the force of contraction to confirm the reactivity of each preparation. After repeating the same procedure once again, the preparations were additionally equilibrated by replacing the nutrient solution twice every 20 minutes. Each drug was administered cumulatively followed by observation of the reaction. Drug action was evaluated by determining the rate of change caused by the drug by taking the difference between the maximum reaction induced by the second administration of isoproterenol and that before drug administration to be 100%, and then calculating the EC₃₀ value as the concentration at which drug action was 30% with respect to force of contraction based on this rate of change.

Those results are shown in Table 5.
i) Action on isolated cardiac muscle of guinea-pig Compound A did not increase heart rate or contraction force to 30% or more at concentrations up to 30 µmol/L. On the other hand, the EC₃₀ values with respect to the number of beats and force of contraction of milrinone were 11.7 µmol/L and 33.9 µmol/L, respectively, and effects on the heart were observed.

When compared with the action on the corpus cavernosum in Experimental example 2, in contrast to the action on the heart of milrinone being about 1/2 the action on the corpus cavernosum, the action on the heart of Compound A was less than 1/90 the action on the corpus cavernosum.

**Table 5**

| | EC₃₀ (µmol/L) | |
|---|---|---|
| | Heart rate | Coronary contraction force |
| Compound A | >30 | >30 |
| Milrinone | 11.7 | 33.9 |

The following findings were determined based on the above experimental results.

Since the pyridazinone compound (I) according to the present invention increased intracavernous pressure during intracavernous injection to anesthetized dogs and exhibited relaxation on isolated corpus cavernosum of rabbit, it was suggested to be effective for treatment of erectile dysfunction.

Synergistic effects resulting from concomitant use of the PDE III inhibitor, milrinone, and the PED V inhibitor, zaprinast, were observed in anesthetized dogs. On the basis of this finding, the possibility was suggested that both inhibitory activity on PDE III and PDE V are involved in the potent action of the pyridazinone compound (I) according to the present invention that causes an increase in intracavernous pressure. Moreover, it was also clearly demonstrated that concomitant therapy with PDE III and PDE V inhibitors results in a considerable improvement in efficacy.

Since the pyridazinone compound (I) according to the present invention is less susceptible to the effect of the nitrogen monoxide synthesis inhibitor, L-NAME, than the PDE V inhibitor, zaprinast, the possibility was also suggested that it is effective for psychogenic erectile dysfunction for which PDE V inhibitors are minimally effective.

Although the action of the pyridazinone compound (I) according to the present invention is enhanced in the corpus cavernosum during concomitant use with the nitrogen monoxide donor, SNP, which has the ability to activate guanylate cyclase, since enhanced effects are not observed in the aorta, the possibility was suggested that it is able to relieve adverse side effects attributable to excessive lowering of blood pressure associated with PDE V inhibitors.

The pyridazinone compound (I) according to the present invention was observed to have hardly any effect on the heart, and effects on the heart, such as increased heart rate and force of contraction as are observed with PDE III inhibitors, are considered to be mild.

### Industrial applicability

The pyridazinone compound (I) according to the present invention has both PDE III and PDE V inhibitory activity, is useful as a remedy agent for erectile dysfunction, and has improved usefulness as compared with therapeutic methods of the prior art.

## Claims

1. A remedial agent for erectile dysfunction containing a 3(2H)-pyridazinone derivative represented by a formula (I): 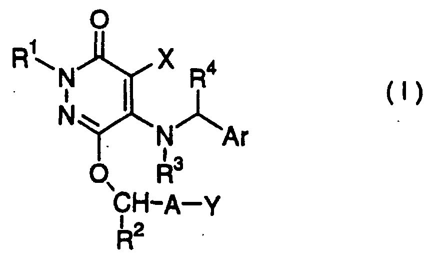
wherein R¹ represents a hydrogen atom, a C₁₋₄ alkyl group, a C₃₋₄ alkenyl group or (CH₂)ₙCO₂R⁵, n represents an integer of 1 to 4 and R⁵ represents a hydrogen atom or a C₁₋₄ alkyl group;
R² represents a hydrogen atom or a C₁₋₄ alkyl group;
R³ and R⁴ each independently represents a hydrogen atom or a C₁₋₃ alkyl group;
A represents a single bond or a straight or branched C₁₋₁₁ alkylene in which a carbon atom on the straight chain may be substituted by one OR² group where R² has the same meaning as defined above;
X represents a chlorine atom, a bromine atom, a hydrogen atom or a cyano group;
Y represents any one of the following (1) to (13):
(1) CO₂R⁵ where R⁵ has the same meaning as defined above,
(2) a cyano group, (3) OR⁶ where R⁶ represents a hydrogen atom, a C₁₋₄ alkyl group or a phenyl group, (4) a thienyl group, (5) a pyridyl group, 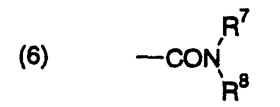
wherein R⁷ and R⁸ each independently represents a hydrogen atom, a C₁₋₄ alkyl group, a C₃₋₈ cycloalkyl group, a phenyl group, a thiazolyl group or a thiadiazolyl group, or R⁷ and R⁸ are combined together to form a straight or branched C₂₋₈ alkylene which may be substituted by a C₁₋₃ alkyl group or a phenyl group or to form a morpholine ring with the nitrogen atom, 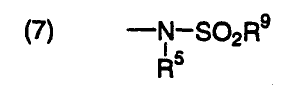 wherein R⁵ has the same meaning as defined above and R⁹ represents a phenyl group which may be substituted by a hydrogen atom, a C₁₋₄ alkyl group or a halogen atom, 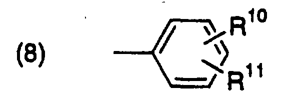 wherein R¹⁰ and R¹¹ each independently represents a hydrogen atom, a halogen atom, a C₁₋₄ alkyl group, a C₁₋₄ acylamino group, OR⁵ where R⁵ has the same meaning as defined above, NHSO₂R⁹ where R⁹ has the same meaning as defined above, or S(O)ₘ-R¹² where m is an integer of 0 to 2 and R¹² represents a C₁₋₄ alkyl group, 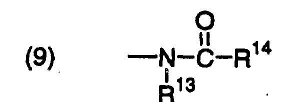 wherein R¹³ represents a hydrogen atom, R¹⁴ represents a phenyl group or R¹³ and R¹⁴ together form C₂₋₈ alkylene which may be substituted by a straight C₁₋₃ alkyl group, 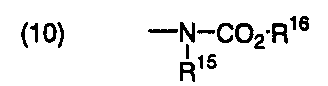 wherein R¹⁵ represents a hydrogen atom or a C₁₋₄ alkyl group, R¹⁶ represents a C₁₋₄ alkyl group or R¹⁵ and R¹⁶ are combined together to form C₂₋₈ alkylene which may be substituted by a straight C₁₋₃ alkyl group, 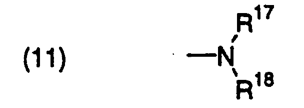 wherein R¹⁷ and R¹⁸ each independently represents a C₁₋₄ alkyl group or R¹⁷ and R¹⁸ are combined together to form C₂₋₈ alkylene which may be substituted by a straight C₁₋₃ alkyl group, 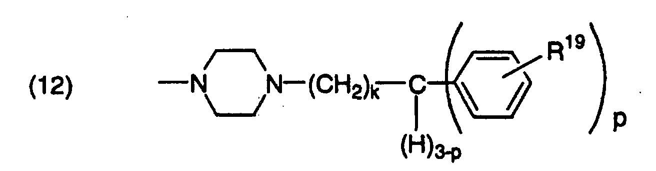 wherein p is 1 or 2, k is an integer of 0 to 3 and R¹⁹ represents a hydrogen atom or a halogen atom, or 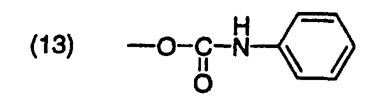
Ar represents any one of the following (1) to (5): 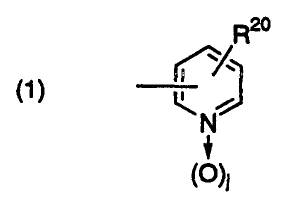 wherein j is 0 or 1, R²⁰ represents a hydrogen atom, a halogen atom or OR¹² where R¹² has the same meaning as defined above, 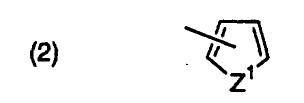 wherein Z¹ represents an oxygen atom or a sulfur atom, 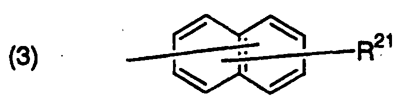 wherein R²¹ represents a hydrogen atom or OR⁵ where R⁵ has the same meaning as defined above, 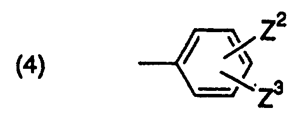 wherein Z² and Z³ each independently represents a hydrogen atom, a halogen atom, a C₁₋₄ alkyl group, OR²² wrere R²² represents a hydrogen atom or a C₁₋₈ alkyl group, O-A¹-Y¹ where A¹ represents a straight or branched C₁₋₈ alkylene and Y¹ represents a C₁₋₄ alkyl group or a phenyl group which may be substituted by a halogen atom, CO₂R⁵ where R⁵ has the same meaning as defined above, or 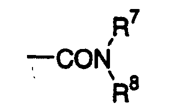 wherein R⁷ and R⁸ have the same meanings as defined above or 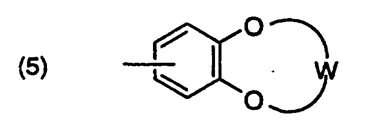 wherein W forms C₁₋₈ alkylene which may be substituted by a straight C₁₋₃ alkyl group,
or a pharmaceutically acceptable salt thereof as an active ingredient.

2. The remedial agent for erectile dysfunction according to Claim 1, wherein R¹ is a hydrogen atom, X is a chlorine atom or a bromine atom and R³ and R⁴ are both hydrogen atoms.

3. The remedial agent for erectile dysfunction according to Claim 1 or 2, wherein Ar is a pyridyl group, a N-oxypyridyl group or a phenyl group represented by the formula: 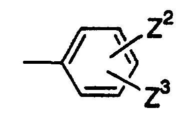 wherein Z² and Z³ each independently represents a hydrogen atom, a halogen atom, a C₁₋₄ alkyl group or OR²² where R²² represents a hydrogen atom or a C₁₋₈ alkyl group.

4. The remedial agent for erectile dysfunction according to any one of Claims 1 to 3, wherein Y is a carbamoyl group represented by the formula: 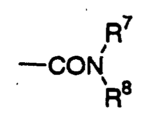 wherein R⁷ and R⁸ each independently represents a hydrogen atom, a C₁₋₄ alkyl group, a C₃₋₈ cycloalkyl group or a phenyl group or R⁷ and R⁸ are combined together to form C₂₋₈ alkylene which may be substituted by a C₁₋₃ alkyl group or a phenyl group or to form a morpholine ring with the nitrogen atom,
or a phenyl group represented by the formula: 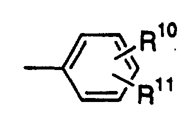 wherein R¹⁰ and R¹¹ each independently represents a hydrogen atom, a halogen atom, a C₁₋₄ alkyl group, a C₁₋₄ acylamino group, OR⁵ where R⁵ represents a hydrogen atom or a C₁₋₄ alkyl group, NHSO₂R⁹ where R⁹ represents a phenyl group which may be substituted by a hydrogen atom, a C₁₋₄ alkyl group or a halogen atom, or S(O)ₘ-R¹² where m is an integer of 0 to 2 and R¹² represents a C₁₋₄ alkyl group.

5. The remedial agent for erectile dysfunction according to Claim 3, wherein Ar is a pyridyl group, a N-oxypyridyl group or a phenyl group which may be substituted by OR²² where R²² represents a C₁₋₄ alkyl group.

6. The remedial agent for erectile dysfunction according to any one of Claims 1 to 5, wherein A is a straight or branched C₁₋₈ alkylene in which a carbon atom on the straight chain may be substituted by one hydroxyl group (-OH).

7. The remedial agent for erectile dysfunction according to any one of Claims 4 to 6, wherein Y is a phenyl group represented by the formula: . 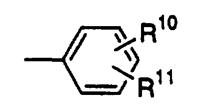 wherein R¹⁰ and R¹¹ each independently represents a hydrogen atom, a halogen atom, a C₁₋₄ alkyl group, a C₁₋₄ acylamino group, OR⁵ where R⁵ represents a hydrogen atom or a C₁₋₄ alkyl group, NHSO₂R⁹ where R⁹ represents a phenyl group which may be substituted by a hydrogen atom, a C₁₋₄ alkyl group or a halogen atom, or S(O)ₘ-R¹² where m is an integer of 0 to 2 and R¹² represents a C₁₋₄ alkyl group.

8. The remedial agent for erectile dysfunction according to Claim 7, wherein Y is a phenyl group which may be substituted by a halogen atom.

9. The remedial agent for erectile dysfunction according to any one of Claims 5 to 8, wherein Ar is a 3-pyridyl group.

10. The remedial agent for erectile dysfunction according to Claim 1, wherein the compound represented by the formula (I) is 4-bromo-6-[3-(4-chlorophenyl)propoxy]-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone or a pharmaceutically acceptable salt thereof.
